# EUROPEAN PATENT SPECIFICATION

(11) **EP 1 457 182 B1**
(45) Date of publication and mention of the grant of the patent: **05.11.2008**
(21) Application number: 04445028.6
(22) Date of filing: 12.03.2004
(51) Int. Cl.: A61F 11/12

(54) **Hearing protector**
Gehörschutzvorrichtung
Dispositif de protection auditive

(30) Priority: 14.03.2003 SE 0300710
(43) Date of publication of application: 15.09.2004
(73) Proprietor: Sordin AB, 79160 Falun (SE)
(72) Inventor: Sjökvist, Ingvar, 14144 Huddinge (SE)
(74) Representative: Fritzon, Rolf

(56) References cited:
- US-A- 997 673
- US-A- 4 023 642
- US-A- 6 095 146
- US-B1- 6 382 213
- US-B1- 6 456 199

## Description

### TECHNICAL FIELD

The present invention relates to an arrangement for preventing or at least reducing high acoustic pressure levels reaching the tympanic membrane of an ear. In particular, the present invention relates to an arrangement for preventing or at least reducing high acoustic pressure levels reaching the tympanic membrane of an ear by means of placing a protector in an external auditory canal of said ear.

### BACKGROUND OF THE INVENTION

In today's environment, human beings and animals live under a constant bombardment of noise. The noise levels, or the acoustic pressure measured in decibels, are often extremely high, so high in fact that they can damage hearing.

Impaired hearing can be caused by sudden loud noises, or from having been exposed to high levels of noise for a relatively long time. Noise with an acoustic pressure of over 80-85 dB is usually considered to cause impaired hearing if the ear is exposed to these levels for quite some time.

To avoid damage to hearing in environments where there are high noise levels, a large number of different ear protectors have been developed. In principle, there are two different types of ear protectors, the first type surrounding the whole of the pinna, and the second type being inserted into the external auditory canal and closing it.

The second type often requires shaping it with the fingers before inserting it into the ear. This means that it is necessary to use both hands to apply the ear protector and also that dirt and bacteria on the hands and fingers risk getting into the ear. For workers who use relatively thick protective gloves, it is difficult to apply the protector without taking off said gloves.

Others require screwing the protector in, or gripping the pinna in order to more easily insert the protector.

An example of a prior art solution requiring screwing the protector into the ear is diclosed in US 997,673.

US 6,382,213 discloses another example of a prior art solution, comprising a combined eyesight and hearing safety apparatus. The hearing protection assembly comprises an arm member and leg member which pivot rotationally around a pivoting attachment. A user wearing the assembly has to pivot the leg member in order to place an earplug into his ear, which can be difficult without taking off gloves, if such are used, or without using both hands.

US 6,095,146 discloses a hearing protecting device, similar, to the eysight and hearing safety apparatus of the above document.

There is therefore a need for an ear protector which is easy to apply in the external auditory canal of an ear and which provides good protection against high levels of noise.

For ear protectors of the first type, total enclosure of the pinna may feel uncomfortable and awkward. A tight arrangement like this can be uncomfortable especially in warm weather.

### DISCLOSURE OF THE INVENTION

The main object of the present invention is therefore to make available an ear protector which prevents or at least reduces the abovementioned problems.

A particular object of the present invention is to make available a method and an apparatus which is comfortable, gives good protection against high levels of noise and is easy to control in the sense of whether protection is required or not at a given time.

The abovementioned objects are achieved by a method and equipment in accordance with the attached independent claims.

Advantageous alternatives and embodiments of the present invention are afforded by means of methods and equipment according to the atached dependent claims.

An advantage of the present invention is that it is possible to remove the ear protector by a simple manoeuvre and then reapply the protector by a simple manoeuvre.

Further features and advantages of the invention will become clear from the following detailed description of embodiments according to the invention.

### BRIEF DESCRIPTION OF THE DRAWINGS

The present invention will be better understood from the following detailed description of preferred embodiments according to the present invention and from attached Figures 1 to 3, which are intended only to illustrate the preferred embodiments and not intended to be restrictive for the present invention.

Figure 1 shows a preferred embodiment of the invention in a perspective view.

Figure 2 shows the arrangement from Figure 1 in a side view.

Figure 3 shows a detail of the arrangement from Figure 1.

### PREFERRED EMBODIMENTS

The description below details specific techniques and applications in order to provide a clear understanding of the present invention. However, it will be obvious to the person skilled in the art that the present invention can be implemented in other embodiments which differ from what has been indicated in detail herein. Detailed descriptions of well known methods and equipment have been omitted so as not to fill the description of the present invention with unnecessary details.

Figure 1 shows a perspective view of a preferred embodiment according to the invention. The arrangement consists of a first part 101, which is a bow intended to fit round the head of a person wearing the ear protector, and a second part 102 and a third part 103. The second and third parts 102 and 103 are intended to bear against the skull around and behind an ear. The second and third parts 102 and 103 secure the ear protector in the right position on the skull.

In an alternative embodiment, the first part 101 is pretensioned by a spring and the second and third parts consist of plates which, with the aid of the spring pretensioning, press on opposite sides of the head in order in this way to secure the ear protector.

A fourth part 104 and a fifth part 105 are arranged pivotably on the ear protector and are shown in Figure 1 in a first, outward position. When a person is wearing the ear protector and the fourth and fifth parts are in the first position, the ear protector does not suppress noise, and, for example, the person is therefore able to talk with someone.

The fourth and fifth parts comprise ear plugs 106 and 107 which are exchangeable and which, when the fourth and/or fifth part assume(s) a second position, fit into the auditory canal of a respective ear. The ear plugs thus suppress noise and the ear protector protects the wearer from high acoustic pressures. The ear plugs can have a number of different designs.

The fourth and fifth parts function independently of one another. Thus, a wearer can choose to protect just one ear from loud noise, for example if noises are coming from a specific direction.

With the design according to the invention, it is possible for the wearer, without taking the actual ear protector off, to cut out the noise suppression by moving the fourth and/or fifth parts to the first position. In this way the user can, for example, communicate with co-workers.

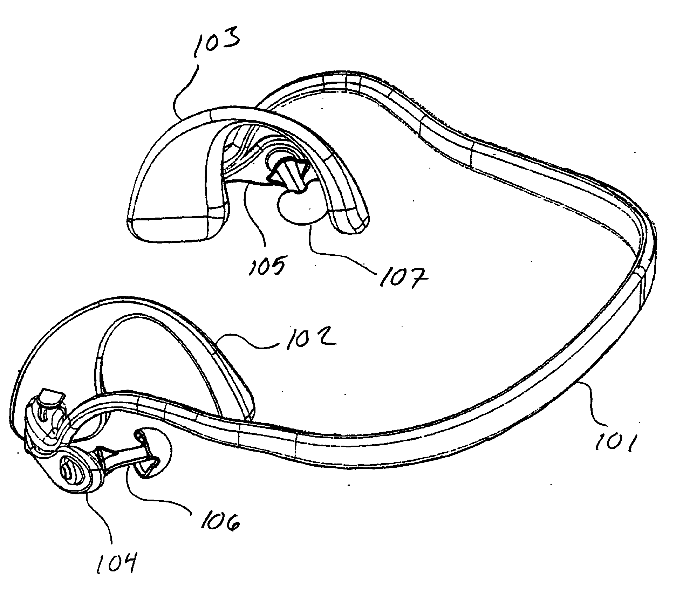

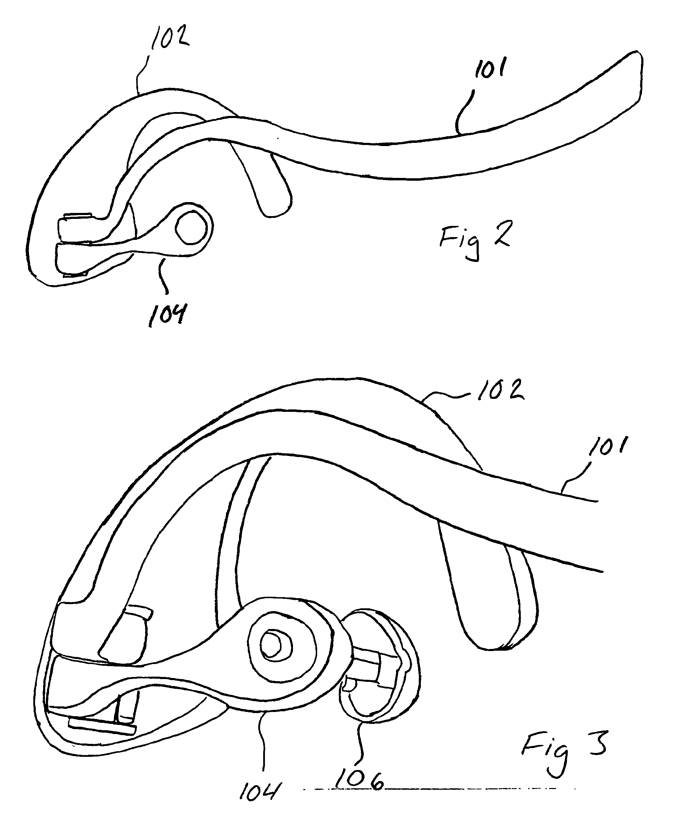

Figure 2 shows the arrangement from Figure 1 in a side view. The same details are indicated by the same reference numbers. Figure 3 shows an enlarged detail of part of the ear protector from Figure 1 in a perspective view when the fourth part 104 is situated in the second, inward position.

The fourth and fifth parts can be pretensioned by a spring so that the ear plug is pressed in towards the auditory canal when the fourth and fifth parts are situated in the second, inward position.

According to a further embodiment of the invention, the fourth and fifth parts are pretensioned by a spring so that a slight pressure on said fourth or fifth part, or on an arrangement which triggers the spring, releases the fourth and fifth parts so that these assume the first, outward position.

It is obvious that the present invention can be varied in many different ways. Such variations are not to be regarded as a departure from the scope of the present invention. All such modifications which are obvious to the skilled person are intended to be included in the scope of the attached patent claims.

## Claims

1. Ear protector arranged to protect a first ear and a second ear from loud noise, comprising a bow which has a first part (101) connecting a second part (102) and a third part (103),
- said second part (102) comprising at least a first contact point to be applied in proximity to said first ear,
- said third part (103) comprising at least a first contact point to be applied in proximity to said second ear,
- the ear protector comprises a fourth part (104) arranged rotatably on said second part (102) and comprising a first ear plug (106) which fits into said first ear to suppress noise,
- the ear protector comprises a fifth part (105) arranged rotatably on said third part (103) and comprising a second ear plug (107) which fits into said second ear to suppress noise,
**characterised in that**
- said fourth part (104) is designed to be able to assume a first position in which said ear plug is in a position outside said first ear and a second position in which said ear plug is in a position which fits in the auditory canal of said first ear and in which position a pressure is exerted inwards towards said auditory canal in order thereby to suppress noise,
- said fifth part (105) is designed to be able to assume a first position in which said ear plug is in a position outside said second ear, and a second position in which said ear plug is in a position which fits in the auditory canal of said second ear and in which position a pressure is exerted inwards towards said auditory canal in order thereby to suppress noise.

2. Ear protector according to claim 1, in which
- said second and third parts (102, 103) are arranged to pass round the pinna of an ear and in this way hold said ear protector securely in a position such that said fourth and fifth parts (104, 105), in the respective second position, fit said ear plugs (106, 107) into said auditory canals.

3. Ear protector according to claim 1, in which
- said first part (101) is pretensioned by a spring so that said second and third parts (102, 103) press towards a position in proximity to said ears in order to hold said ear protector securely in a position such that said fourth and fifth parts (104, 105), in the respective second positions, fit said ear plugs (106, 107) into said auditory canals.

4. Ear protector according to claim 1, in which
- said fourth and fifth parts (104, 105) are pretensioned by a spring so that, in said second position, they exert a pressure inwards towards said auditory canal.

5. Ear protector according to claim 1, in which
- said fourth and fifth parts (104, 105) are pretensioned by a spring in said second position so as to assume said first position under a slight pressure.

6. Ear protector according to claim 1, in which
- said ear protector comprises a radio, mobile telephone, pager or other electronic equipment and devices for conveying sound or a signal in an audible manner through said ear plugs.

7. Ear protector according to claim 1, in which
- said ear plugs (106, 107) can be exchanged from said fourth and fifth parts (104, 105).

## Patentansprüche

1. Gehörschutzvorrichtung zum Schützen eines ersten Ohrs und eines zweiten Ohrs vor lautem Lärm, umfassend einen Bügel mit einem ein zweites Teil (102) und ein drittes Teil (103) miteinander verbindenden ersten Teil (101),
- wobei das zweite Teil (102) mindestens eine in der Nähe des ersten Ohrs anzulegende erste Kontaktstelle umfasst,
- wobei das dritte Teil (103) mindestens eine in der Nähe des zweiten Ohrs anzulegende erste Kontaktstelle umfasst,
- die Gehörschutzvorrichtung ein drehbar am zweiten Teil (102) angeordnetes viertes Teil (104) umfasst, das einen ersten Gehörschutzstöpsel (106) umfasst, der in das erste Ohr passt, zur Unterdrückung von Lärm,
- die Gehörschutzvorrichtung ein drehbar am dritten Teil (103) angeordnetes fünftes Teil (105) umfasst, das einen zweiten Gehörschutzstöpsel (107) umfasst, der in das zweite Ohr passt, zur Unterdrückung von Lärm,
**dadurch gekennzeichnet, dass**
- das vierte Teil (104) dazu ausgelegt ist, eine erste Position, in der sich der Gehörschutzstöpsel an einer Position außerhalb des ersten Ohrs befindet, und eine zweite Position einnehmen zu können, in der sich der Gehörschutzstöpsel in einer Position befindet, die in den Hörkanal des ersten Ohrs passt, und in der nach innen in Richtung des Hörkanals ein Druck ausgeübt wird, um **dadurch** Lärm zu unterdrücken,
- das fünfte Teil (105) dazu ausgelegt ist, eine erste Position, in der sich der Gehörschutzstöpsel an einer Position außerhalb des zweiten Ohrs befindet, und eine zweite Position einnehmen zu können, in der sich der Gehörschutzstöpsel in einer Position befindet, die in den Hörkanal des zweiten Ohrs passt, und in der nach innen in Richtung des Hörkanals ein Druck ausgeübt wird, um **dadurch** Lärm zu unterdrücken.

2. Gehörschutzvorrichtung nach Anspruch 1, bei der
- das zweite und dritte Teil (102, 103) so angeordnet sind, dass sie um die Ohrmuschel eines Ohrs herumlaufen und auf diese Weise die Gehörschutzvorrichtung sicher festhalten, so dass das vierte und fünfte Teil (104, 105), in der jeweiligen zweiten Position, die Gehörschutzstöpsel (106, 107) in die Hörkanäle passen.

3. Gehörschutzvorrichtung nach Anspruch 1, bei der
- das erste Teil (101) von einer Feder so vorgespannt ist, dass das zweite und dritte Teil (102, 103) gegen eine in der Nähe der Ohren liegenden Position drücken, um die Gehörschutzvorrichtung sicher derart in einer Position festzuhalten, dass das vierte und fünfte Teil (104, 105), in der jeweiligen zweiten Position, die Gehörschutzstöpsel (106, 107) in die Hörkanäle passen.

4. Gehörschutzvorrichtung nach Anspruch 1, bei der
- das vierte und fünfte Teil (104, 105) von einer Feder so vorgespannt sind, dass sie, in der zweiten Position, nach innen in Richtung des Hörkanals einen Druck ausüben.

5. Gehörschutzvorrichtung nach Anspruch 1, bei der
- das vierte und fünfte Teil (104, 105) von einer Feder so in der zweiten Position vorgespannt sind, dass sie die erste Position unter einem geringen Druck einnehmen.

6. Gehörschutzvorrichtung nach Anspruch 1, bei der
- die Gehörschutzvorrichtung ein Radio, Mobiltelefon, Pager oder anderes elektronisches Gerät und Einrichtungen zum Übermitteln von Geräuschen oder einem Signal in hörbarer Weise durch die Gehörschutzstöpsel umfasst.

7. Gehörschutzvorrichtung nach Anspruch 1, bei der
- die Gehörschutzstöpsel (106, 107) an dem vierten und fünften Teil (104, 105) ausgetauscht werden können.

## Revendications

1. Dispositif de protection auditive conçu pour protéger une première oreille et une deuxième oreille d'un bruit de forte intensité, comprenant un élément recourbé comportant une première partie (101) reliant une deuxième partie (102) et une troisième partie (103),
- ladite deuxième partie (102) comprenant au moins un premier point de contact destiné à être appliqué à proximité de ladite première oreille,
- ladite troisième partie (103) comprenant au moins un premier point de contact destiné à être appliqué à proximité de ladite deuxième oreille,
et comprenant :
- une quatrième partie (104) agencée rotative sur ladite deuxième partie (102) et comprenant un premier bouchon d'oreille (106) qui s'enfonce dans ladite première oreille pour supprimer le bruit,
- une cinquième partie (105) agencée rotative sur ladite troisième partie (103) et comprenant un deuxième bouchon d'oreille (107) qui s'enfonce dans ladite deuxième oreille pour supprimer le bruit,
**caractérisé en ce que** :
- ladite quatrième partie (104) est conçue pour pouvoir prendre une première position dans laquelle ledit bouchon d'oreille occupe une position extérieure à ladite première oreille, 1 et une deuxième position dans laquelle ledit bouchon d'oreille occupe une position enfoncée dans le conduit auditif de ladite première oreille et dans laquelle une pression s'exerce vers l'intérieur en direction dudit conduit auditif de manière à supprimer le bruit,
- ladite cinquième partie (105) est conçue pour pouvoir prendre une première position dans laquelle ledit bouchon d'oreille occupe une position extérieure à ladite deuxième oreille, et une deuxième position dans laquelle ledit bouchon d'oreille occupe une position enfoncée dans le conduit auditif de ladite deuxième oreille et dans laquelle une pression s'exerce vers l'intérieur en direction dudit conduit auditif de manière à supprimer le bruit.

2. Dispositif de protection auditive selon la revendication 1,
- lesdites deuxième et troisième parties (102, 103) étant conçues pour passer autour du pavillon d'une oreille de manière à maintenir fixement ledit dispositif de protection auditive dans une position telle que lesdites quatrième et cinquième parties (104, 105), occupant la deuxième position respective, enfoncent lesdits bouchons d'oreille (106, 107) dans lesdits conduits auditifs.

3. Dispositif de protection auditive selon la revendication 1,
- ladite première partie (101) étant précontrainte par un ressort de façon à comprimer lesdites deuxième et troisième parties (102, 103) en direction d'une position à proximité desdites oreilles de manière à maintenir fixement ledit dispositif de protection auditive dans une position telle que lesdites quatrième et cinquième parties (104, 105), occupant la deuxième position respective, enfoncent lesdits bouchons d'oreille (106, 107) dans lesdits conduits auditifs.

4. Dispositif de protection auditive selon la revendication 1,
- lesdites quatrième et cinquième parties (104, 105) étant précontraintes par un ressort de façon à exercer, dans ladite deuxième position, une pression vers l'intérieur en direction dudit conduit auditif.

5. Dispositif de protection auditive selon la revendication 1,
- lesdites quatrième et cinquième parties (104, 105) étant précontraintes dans ladite deuxième position par un ressort de façon à prendre ladite première position sous une légère pression.

6. Dispositif de protection auditive selon la revendication 1, comprenant une radio, un téléphone mobile, un récepteur de radiomessagerie ou d'autres appareils et dispositifs électroniques pour transmettre de façon audible du son ou un signal à travers lesdits bouchons d'oreille.

7. Dispositif de protection auditive selon la revendication 1,
- lesdits bouchons d'oreille (106, 107) pouvant être substitués desdites quatrième et cinquième parties (104, 105).
